# EUROPEAN PATENT APPLICATION

(11) **EP 3 069 734 A1**
(43) Date of publication of application: **21.09.2016**
(21) Application number: 15159327.4
(22) Date of filing: 17.03.2015
(51) Int. Cl.: A61K 47/48, A61P 35/00, A61K 31/395

(54) **CRYPTOPHYCIN-BASED ANTIBODY-DRUG CONJUGATES WITH NOVEL SELF-IMMOLATIVE LINKERS**

(71) Applicant: Exiris S.r.l., 00182 Roma (IT)
(72) Inventor: Steinkuhler, Christian, 00185 Roma (IT); Gallinari, Paola, 00185 Roma (IT); Osswald, Bianca, 33602 Bielefeld (DE); Sewald, Norbert, 33739 Bielefeld (DE); Ritzefeld, Markus, 33619 Bielefeld (DE); Frese, Marcel, 32130 Enger (DE)
(74) Representative: Spadaro, Marco

(57) **Abstract**

The present Invention relates to antibody- or peptide-drug conjugate compounds where one or more cryptophycin derivatives (macrocyclic depsipeptide) are covalently attached by a self-immolative linker which binds to one or more tumor-associated antigens or cell-surface receptors. The linker contains a cleavage site for proteases and a dipeptide unit able to form a diketopiperazine. These compounds may be useful in methods of diagnosis or treatment of cancer, and other diseases and disorders, such as immune or infective diseases.

## Description

### FIELD OF THE INVENTION

The present invention relates to the field of medicinal chemistry, in particular to compounds with anti-cancer activity and more specifically to antibodies conjugated with chemotherapeutic macrocyclic depsipeptide drugs or toxins. The invention also relates to the above compounds for use in *in vitro, in situ,* and *in vivo* diagnosis or treatment of mammalian cells or subjects affected by cancer, an autoimmune disease, or an infectious disease.

### BACKGROUND OF THE INVENTION

Antibody therapy has been established for the targeted treatment of patients with cancer, immunological and angiogenic disorders. For this purpose, therapeutic monoclonal antibodies (TMAs) have been developed (Firer M. A. J. Hematol. Oncol. 2012, 5, 70; Mullard, A. Nature Rev. Drug Discov. 2013, 12, 329). However, many TMAs that showed promise in preclinical studies when used on their own failed in the clinic because of insufficient cancer cell toxicity.

Monoclonal antibody therapy has been established for the targeted treatment of patients with cancer, immunological and angiogenic disorders. An example of successful antibody therapy is HERCEPTIN^{®} (trastuzumab), a recombinant DNA-derived humanized monoclonal antibody that selectively binds with high affinity to the extracellular domain of the human epidermal growth factor receptor 2 protein, HER2 (ErbB2) (US 5,821,337; US 6,054,297; US 6,407,213; US 6,639,055; Coussens L, et al (1985) Science 230:1132-9; Slamon D J, et al (1989) Science 244:707-12). Although HERCEPTIN is a breakthrough in treating patients with ErbB2-overexpressing breast cancers that have received extensive prior anti-cancer therapy, the majority of the patients in this population fail to respond or respond only poorly to HERCEPTIN treatment.

To overcome such a problem, there has been an exponential growth in the field of antibody-drug conjugates (ADCs), where a monoclonal antibody (mAb, e.g. trastuzumab) is linked to a highly cytotoxic drug. ADCs gain more and more importance in the therapy of cancer. Three ADCs have been approved by the US FDA: Mylotarg® (gemtuzumab ozogamicin, Wyeth Pharmaceuticals), Adcetris® (brentuximab vedotin, Seattle Genetics), and Kadcyla® (ado-trastuzumab emtansin, Roche), while 35 ADCs are currently in clinical trials. Tumor selectivity is displayed by the mAb whereas the linker is responsible for the release of the drug, the stability and the overall solubility under physiological conditions.

Mylotarg® is composed of a hu CD33 antibody linked to calicheamicin and was approved in 2000 for the treatment of acute myeloid leukemia, but withdrawn in 2010. Adcetris® (brentuximab vedotin, Seattle Genetics, Formula 1, below) was approved in 2011 and is composed of monomethyl auristatin E (MMAE) connected to an antibody against CD30, which is expressed in classical Hodgkin lymphoma (HL) and systemic anaplastic large cell lymphoma (sALCL), Kadcyla^{®} (ado-trastuzumab emtansin, Roche, Formula 2, below) was approved in 2013 for treatment of refractory human epidermal growth factor receptor 2 (HER2) positive metastatic or locally advanced breast cancer and contains the monoclonal antibody trastuzumab (Herceptin) linked to the cytotoxic agent mertansine (DM1).

Ado-trastuzumab Emtansin (Kadcyla^{®}) and Brentuximab Vedotin (Adcetris^{®}) are the only two ADCs which are currently approved by the US Food and Drug Administration (FDA) and European Medicine Agency (EMA).

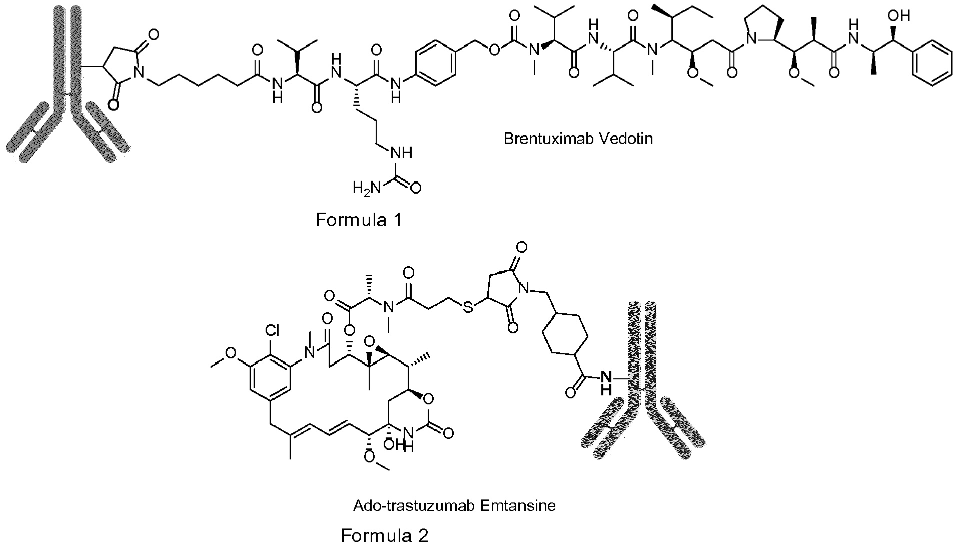

The extraordinary activity of cryptophycins has been acknowledged in some patents lately. Endocyte patented the cryptophycin folate conjugate (Formula 3) equipped with a cleavable disulfide linker. Such folate conjugates target cancer cell lines overexpressing folate receptors (US2009/002993).

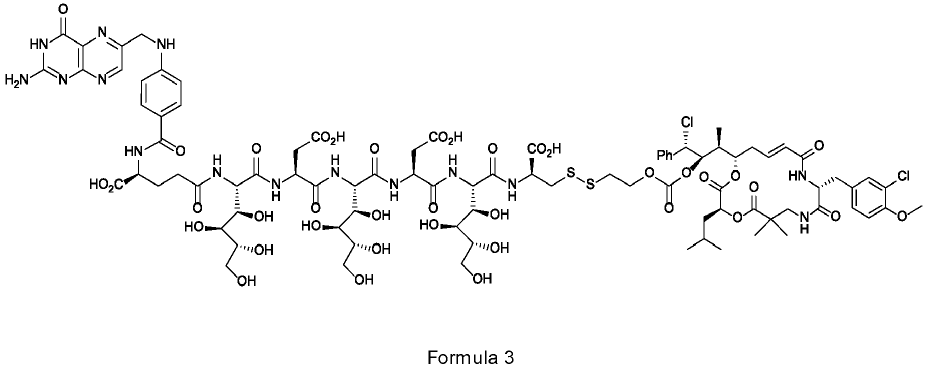

Sanofi-Aventis published two patents disclosing data on cryptophycin antibody-drug conjugates (ADCs) very recently (US2011/001052; FR2947269) Mainly cryptophycin derivatives with a modified aryl substituent were selected to obtain cryptophycins with suitable functionalities (Formula 4).

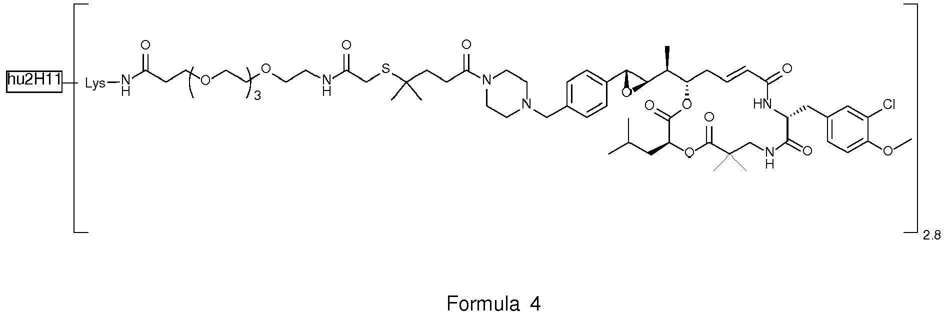

Different linker types, among them a cleavable disulfide-, a PEG-, a thioether- and a thiazole linker were used. In all cases, the linker was connected to hu2H11, a monoclonal antibody targeting the EphA2 receptor. The IC₅₀ values for two cryptophycin ADCs were also reported against the human breast gland cancer cell-line MDA-MB-231. The ADC shown in Formula 4 contains a sterically hindered thioether linker and shows an IC₅₀ value of 0.710 nM. Another ADC contains a sterically hindered disulfide linker (IC₅₀: 0.150 nM). No cytotoxicity results against multi-drug resistant (MDR) cell lines were reported.

Cryptophycins (Formula 5) are a family of antimitotic depsipeptides showing with very high cytotoxicity even against multi-drug resistant (MDR) cancer cells. Cryptophycin-52 displays very high activity against multi-drug resistant (MDR) tumour cell lines. Many MDR tumour cells express P glycoprotein (P-gp), which acts as an efflux pump. As the rational design of P-gp resistant drugs is still difficult, this activity is another valuable feature of cryptophycins.

The first representative was isolated from cyanobacteria Nostoc sp. in 1990. Their bioactivity is based on their interaction with the protein tubulin. Cryptophycins were found to induce apoptosis due to inhibition of the microtubule dynamics. Consequently, cryptophycin analogues are considered as potential antitumor agents.

Cryptophycin-52 (LY355703) passed clinical phase I studies, but subsequent clinical phase II studies were discontinued because of lacking efficacy in vivo, high neurotoxicity, and dose-limiting toxicity at the chosen doses.

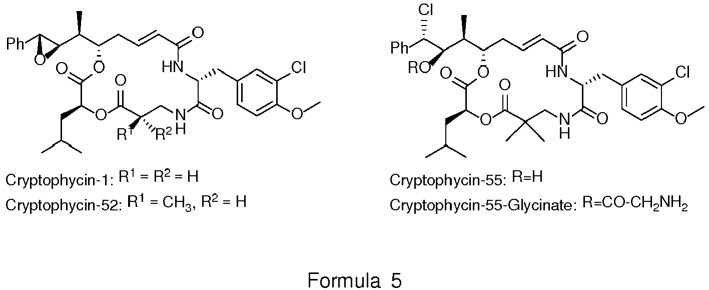

Only few ADCs have been currently approved for the therapy of cancer. A higher diversity of ADCs is necessary to cope with drug resistance.

Up to now no cryptophycin-based ADC has been approved and no cryptophycin-55-glycinate has been described for conjugation to an antibody

All described cryptophycin conjugates are only accessible by long synthetic routes (see for example WO2011/001052). However, Sewald and co-workers described short and efficient synthetic routes (Nat. Prod. Rep. 2013, 30, 924-940).

There is still the strong need to provide drug conjugates, in particular antibody-drug conjugates (also commonly referred as ADCs) and peptide-drug conjugates (also commonly referred as PDCs) for safe use in therapy of diseases, in particular cancer, immunological diseases, infective diseases.

There is also still the strong need to provide drug conjugates, in particular antibody-drug conjugates (also commonly referred as ADCs) and peptide-drug conjugates (also commonly referred as PDCs) for use in diagnostics of diseases, in particular cancer, immunological diseases, infective diseases.

### DESCRIPTION OF THE INVENTION

It has now been found that conjugates where the chlorohydrin cryptophycin-55 is linked to a monoclonal antibody or a peptide across a self-immolative, peptide-derived linker are highly efficient against tumour cells.

The conjugates according to the present invention bind to one or more tumor-associated antigens or cell-surface receptors, therefore, they can be used in the diagnosis or treatment of those disease which can be diagnosed or treated thanks to the binding to tumor-associated antigens or cell-surface receptors.

It is an object of the present invention a conjugate of formula (I) 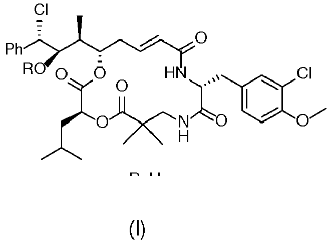 wherein R is -CO-CH₂-X-(A)ₙ-B
wherein X is selected from the group consisting of NR^{a}, wherein R^{a} is selected from a group consisting of H, C₁-C₁₀ alkyl or
X is -O;
A is a self-immolative linker,
n is 0 or 1;
B is selected from the group consisting of a peptide, a polypeptide/protein, and an antibody
and the pharmaceutically acceptable salts thereof.

Another object of the present invention is a pharmaceutical composition comprising a conjugate as above described as active ingredient in admixture with at least one pharmaceutically acceptable vehicle and/or excipient.

Another object of the present invention is the conjugate as above described, for use as a medicament.

Another object of the present invention is the conjugate as above described, for use as a diagnostic.

These and other objects of the present invention will be now described in detail also by means of examples and Figures.

In the Figures:
Figure 1: shows cell viability assay after treatment of HER2-positive SK-BR3 cells with the compounds indicated in the panels.
Figure 2: shows purification of the drug linker-conjugated antibody wherein, peak 2 corresponds to the monomeric form and peak 1 corresponds to an oligomeric form of the cryptophycin-55-glycinate-conjugated antibody (Figure 2, left panel), as assessed by comparison with suitable protein MW standards (not shown) and the purified unconjugated antibody (Figure 2, right panel).

### DETAILED DESCRIPTION OF THE INVENTION

The conjugate according to the present invention can be a drug conjugate with an antibody, preferably a monoclonal antibody, in which case is also briefly referred to as ADC, or with a peptide, polypeptide or protein.

Antibodies, in particular monoclonal antibodies are well known in therapy, in particular for conjugation with a drug.

The conjugate according to the present invention can be a drug conjugate with an antibody, preferably a monoclonal antibody, in which case is also briefly referred to as ADC, or with a peptide, polypeptide or protein, in which case is also briefly referred to as PDC,.

Peptides are well known in therapy, in particular for conjugation with a drug. Peptides, polypeptides or proteins according to the present invention can comprise natural amino acids, either in L- or D- or both configuration, as well as artificial peptides.

In a preferred embodiment of the present invention, in the conjugate, the antibody is a monoclonal antibody, or a nanobody. According to a further embodiment of the present invention, the nanobody is selected from the group consisting of single-domain antibody and camelid antibody).

In a particularly preferred embodiment of the present invention, in the antibody drug conjugate (ADC) the monoclonal antibody is selected from the group consisting of trastuzumab, gemtuzumab, brentuximab, rituximab, cetuximab, panitumumab, ofatumumab, obinutuzumab, pertuzumab.

In a preferred embodiment of the present invention, the peptide B binds to somatostatin receptors. In a particularly preferred embodiment of the present invention, the peptide is selected from the group consisting of octreotide, pasireotide or lanreotide.

In an embodiment of the present invention, in the conjugate, the self-immolative linker A is a moiety of formula (II) 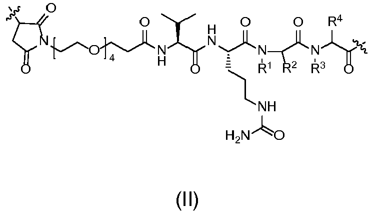 wherein R¹ is selected from the group consisting of H, (C₁-C₁₀) alkyl R² optionally together with R¹, is the residue of an amino acid side chain, R³ is selected from the group consisting of H, (C₁-C₁₀) alkyl R⁴, optionally together with R³ is the residue of an amino acid side chain.

In another embodiment of the present invention, the conjugate has formula (III) 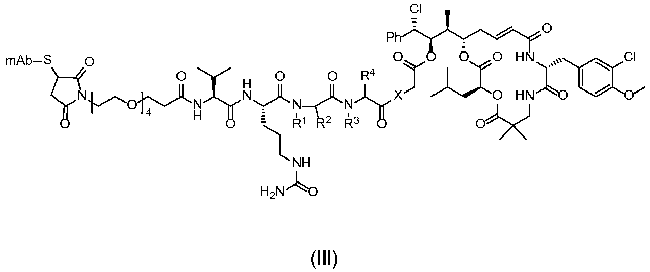 wherein R¹, R², R³ and R⁴ are as defined above, X is selected from the group consisting of NH, N- (C₁-C₁₀)- Alkyl, O; mAb represents a monoclonal antibody or a nanobody, or a peptide or a polypeptide.

In another embodiment of the present invention, the conjugate has formula (IV)

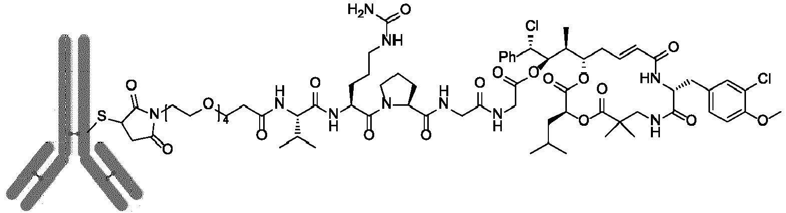

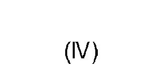

In another embodiment of the present invention, the conjugate has formula (V) 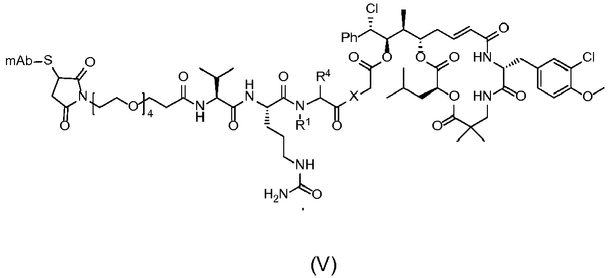 wherein R¹ is selected from the group consisting of H, (C₁-C₁₀) alkyl R⁴, optionally together with R¹, is the residue of an amino acid side chain; X is selected from the group consisting of NH, N- (C₁-C₁₀), Alkyl O; mAb represents a monoclonal antibody, a peptide or a polypeptide.

In an embodiment of the present invention, the conjugate is for use for the therapeutic treatment of a disease selected from the group consisting of cancer or an autoimmune disease or an infective disease.

In an embodiment of the present invention, the conjugate is for use for the diagnosis of a disease selected from the group consisting of cancer or an autoimmune disease or an infective disease. Other than *in vivo,* the conjugates according to the present invention can be used also for an *in vitro* or *in situ* diagnosis.

In a preferred aspect of the present invention, cryptophycin-55 glycinates or cryptophycin glycolates are used. They show cytotoxicity, which is not substantially lower than that of cryptophycin-55 (Table I) and, additionally, have the benefit of a functional group which is suitable for conjugation. Via this function, cryptophycin-55-glycinate or cryptophycin-55-glycolate can be conjugated to a monoclonal antibody (trastuzumab shows high selectivity towards the HER2-receptor which is overexpressed in 20-25% of all breast tumors) via a protease cleavable, self-immolative linker.

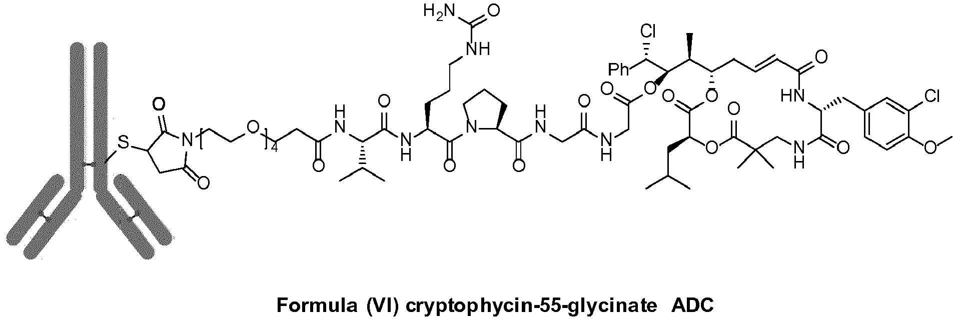

Self immolative linkers are well-known in the design of drug-conjugates, in particular antibody-drug conjugates (ADC) and peptide-drug conjugates, see for example US 6214345 and the related references; Carl P.L. et al. (1982). The linker is composed of an attachment site (maleimide, active ester, etc.) which is reactive for functional groups present in proteins (thiols, amines, etc.), a hydrophilic spacer (e.g. ethylene glycol based), a protease cleavage site (e.g. Val-Cit), and a dipeptide (Scheme 2) or tripeptide unit (Scheme 1) able to release cryptophycin-55, cryptophycin-55 glycinate, cryptophycin-55 glycolate, or other peptidomimetic cryptophycin-55 derivatives. Cryptophycin-55 is assumed to be converted to cryptophycin-52.

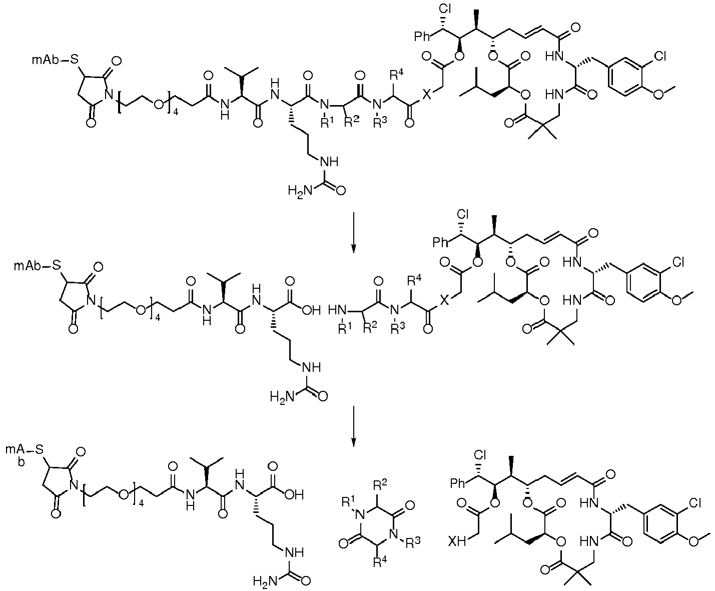

Scheme 1. Cleavage by a protease releases a diketopiperazine and a cryptophycin-55 derivative (e.g. glycinate) (R¹ = H, Alkyl, R² = any amino acid side chain, R³ = H, Alkyl, R⁴ = any amino acid side chain, X = NH, NAlkyl, O)

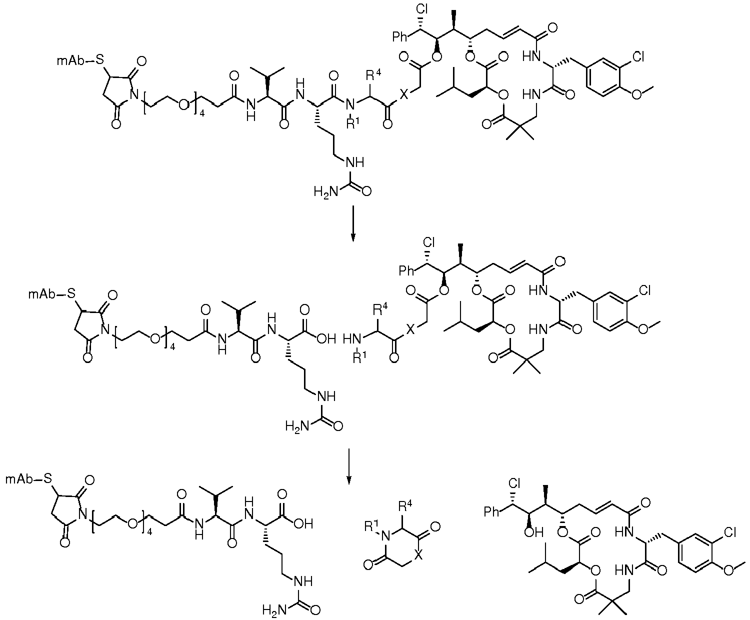

Scheme 2. Cleavage by a protease releases a diketopiperazine (X = NH or NAlkyl) or diketomorpholine (X =O) and cryptophycin-55 (R¹ = H, Alkyl, R⁴ = any amino acid side chain, X = NH, NAlkyl, O)

The conjugate of the present invention can be prepared according to conventional techniques well known in the art.

For example, Cryptophycin-52 can be prepared according to a route published by Weiss C, et al (2012) Beilstein Journal of Organic Chemistry 8:2060-6 and Weiss C, et al (2013) Natural Product Reports 30:924-40.

Cryptophycin-55 can be prepared starting from Cryptophycin-52, for example by opening the oxirane ring of Cryptophycin-52. Opening of an oxirane ring in order to introduce a halogen atom is well within the knowledge of the skilled person. For example hydrochloric acid can be used. Reaction media are usually organic solvents suitable for this kind of reactions, as well as reaction conditions and working up of the reaction and isolation of the desired product.

Cryptophycin-55-glycinate can also be prepared according to well-known synthetic methods, for example through the corresponding trifluoroacetate (see Liang J, et al (2005) Investigational New Drugs 213-224).

Self-immolative linkers suitable for the present invention are also well-known as well as their preparation.

In a preferred embodiment of the present invention, *tert*-Butyl-15-hydroxy-4,7,10,13-tetraoxapentadecanoate can be synthesized according to a procedure published in Seitz O, et al (1997) Journal of Organic Chemistry 62:813-826. *tert*-Butyl-15-maleimido-4,7,10,13-tetraoxapentadecanoate can be synthesized according to Warnecke A, (2002) Dissertation. 15-Maleimido-4,7,10,13-tetraoxapentadecanoic acid can be prepared according to Warnecke A, (2002) Dissertation.

In a preferred embodiment of the present invention, maleimide-PEG-Val-Cit-Pro-Gly can be synthesized according to standard Fmoc SPPS [Fields G and Noble R L, (2009) International Journal of Peptide and Protein Research 33,3:161-214].

Other well-known self-immolative linkers can be used in the present invention.

Thereafter, Cryptophycin-55-glycinate Peptide can be prepared by the well-known Maleimide conjugation method.

An antibody-drug conjugate (ADC) with cryptophycin-55-glycinate can be prepared according the common knowledge in this field. For example upon partial reduction of the antibody inter-chain disulfide bonds with TCEP (tris(2-carboxyethyl)phosphine) method or the partial reduction of the antibody inter-chain disulfide bonds with 2-MEA (2-Mercaptoethylamine·HCl) method.

Any other suitable method can be conveniently used.

Peptide-drug conjugates (PDC) according to the present invention can also be prepared with conventional methods.

ADCs and PDCs of the present invention can be tested for their efficacy by *in vitro,* for example on selected cell lines recognizing the antibody, for ADCs, or presenting receptors recognized by the peptide for PDCs, or *in vivo* methods using laboratory animal models.

The cryptophycin-55-glycinate ADC according to the present invention shows a much higher cytotoxicity than Trastuzumab on a Her2-positive breast carcinoma cell line (SK-BR3).

Different from cryptophycin-55 and cryptophycin-55-glycinate, the ADC cytotoxic effect is highly selective for the HER2-positive SK-BR3 cell line compared to MCF7, a breast carcinoma cell line with low HER2-expression or HCT116, a colon carcinoma cell line with low HER2-expression.

The cryptophycin-based conjugates according to the present invention offer several advantages:
The cryptophycin derivatives released show cytotoxic effects towards tumor cells already at subnanomolar concentration.

The cryptophycin derivatives released are not a substrate for PgP and therefore resistance against cryptophycins may be lower.

The synthetic route is based on cryptophycin-55-glycinate, a cryptophycin which does not have to undergo chemical modification on its backbone before conjugation

Selective addressing of HER2-overexpressing breast tumor cells is possible via the antibody

The ADC offers an alternative mechanism of drug-liberation with formation of diketopiperazine derivatives or analogues after enzymatic cleavage of the linker

The cryptophycin derivatives can be used for synthesizing different conjugates, thus providing higher diversity of drug conjugates.

The conjugates according to the present invention can be administered by means of a pharmaceutical composition, wherein an effective amount of the conjugate is admixed with at least one pharmaceutically acceptable vehicle and/or at least one pharmaceutically acceptable excipient.

Said compositions are suitable for veterinary or human administration.

The compositions of the present invention can be in any form that allows for the composition to be administered to a subject, either animal or human. For example, the composition can be in the form of a solid, liquid or gas (aerosol). Typical routes of administration include, without limitation, oral, topical, parenteral, sublingual, rectal, vaginal, ocular, and intranasal. Parenteral administration includes subcutaneous injections, intravenous, intramuscular, intrasternal injection or infusion techniques. Preferably, the compositions are administered parenterally, more preferably intravenously. Pharmaceutical compositions of the invention can be formulated so as to allow a conjugate of the present invention to be bioavailable upon administration. Compositions can take the form of one or more dosage units.

Materials used in preparing the pharmaceutical compositions shall be non-toxic in the amounts used.

The dosage of the active ingredient in the pharmaceutical composition depends on a variety of factors, such as for example, the type of subject to be administered (for example human), the particular form of the conjugate of the invention, the manner of administration, and the composition employed.

Pharmaceutically acceptable carriers and vehicles are well known in the art and do not need further description. They can be in solid, for example particulate, form or can be liquid, for example, an oral syrup or injectable liquid. In addition, the carrier or vehicle can be gaseous, so as to provide an aerosol composition useful in, e. g., inhalatory administration.

When intended for oral administration, the composition is preferably in solid or liquid form, where semisolid, semi-liquid, suspension and gel forms are included within the forms considered herein as either solid or liquid.

As a solid composition for oral administration, the composition can be formulated into a powder, granule, compressed tablet, pill, capsule, chewing gum, wafer or the like form. Such a solid composition typically contains one or more inert diluents. In addition, one or more of the following can be present: binders such as carboxymethylcellulose, ethyl cellulose, microcrystalline cellulose, or gelatin; excipients such as starch, lactose or dextrins, disintegrating agents such as alginic acid, sodium alginate, corn starch and the like; lubricants such as magnesium stearate; glidants such as colloidal silicon dioxide; sweetening agents such as sucrose or saccharin, a flavoring agent such as peppermint, methyl salicylate or orange flavoring, and a coloring agent.

When the composition is in the form of a capsule, e. g., a gelatin capsule, it can contain, in addition to materials of the above type, a liquid carrier such as polyethylene glycol, cyclodextrin or a fatty oil.

The composition can be in the form of a liquid, e. g., an elixir, syrup, solution, emulsion or suspension. The liquid can be useful for oral administration or for delivery by injection. When intended for oral administration, a composition can comprise one or more of a sweetening agent, preservatives, dye/colorant and flavor enhancer. In a composition for administration by injection, one or more of a surfactant, preservative, wetting agent, dispersing agent, suspending agent, buffer, stabilizer and isotonic agent can also be included.

The liquid compositions of the invention, whether they are solutions, suspensions or other like form, can also include one or more of the following: sterile diluents such as water for injection, saline solution, preferably physiological saline, Ringer's solution, isotonic sodium chloride, fixed oils such as synthetic mono or digylcerides which can serve as the solvent or suspending medium, polyethylene glycols, glycerin, cyclodextrin, propylene glycol or other solvents; antibacterial agents such as benzyl alcohol or methyl paraben; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. A parenteral composition can be enclosed in ampoule, a disposable syringe or a multiple-dose vial made of glass, plastic or other material. Physiological saline is a preferred adjuvant.

An injectable composition is preferably sterile.

The amount of the conjugate of the invention that is effective in the treatment of a particular disorder or condition will depend on the nature of the disorder or condition, and can be determined by standard clinical techniques, In addition, in vitro or in vivo assays can optionally be employed to help identify optimal dosage ranges. The precise dose to be employed in the compositions will also depend on the route of administration, and the seriousness of the disease or disorder, and should be decided according to the judgment of the practitioner and each patient's circumstances.

The composition according to the present invention comprises an effective amount of a conjugate of the invention such that a suitable dosage will be obtained.

The dosage can be determined by body weight or by body surface of the subject to be administered.

Generally, the dosage is typically about 0.1 mg/kg to about 250 mg/kg of the animal's body weight.

In another embodiment, administration can be by direct injection at the site (or former site) of a tumor or the manifestation of an autoimmune disease.

In another embodiment, the conjugates of the invention can be delivered in a vesicle, in particular a liposome (see Langer, Science 249: 1527-1533 (1990); Treat et al., in Liposomes in the Therapy of Infectious Disease and Cancer, Lopez-Berestein and Fidler (eds. ), Liss, New York, pp. 353-365 (1989); Lopez-Berestein, ibid., pp. 317-327; see generally ibid.)

In yet another embodiment, the conjugates of the invention can be delivered in a controlled release system. In one embodiment, a pump can be used (see Langer, supra; Sefton, CRC Crit. Ref. Biomed. Eng. 14: 201 (1987); Buchwald et al., Surgery 88: 507 (1980); Saudek et al., N. Engl. J. Med. 321: 574 (1989)). In another embodiment, polymeric materials can be used (see Medical Applications of Controlled Release, Langer and Wise (eds.), CRC Press , Boca Raton, Florida (1974); Controlled Drug Bioavailability, Drug Product Design and Performance, Smolen and Ball (eds.), Wiley, New York (1984); Ranger and Peppas, J. Macromol. Sci. Rev. Macromol. Chem. 23: 61 (1983); see also Levy et al., Science 228: 190 (1985); During et al., Ann. Neurol. 25: 351 (1989); Howard et al., J. Neurosurg. 71: 105 (1989)). In yet another embodiment, a controlled-release system can be placed in proximity of the target of the Compounds of the Invention or compositions, e. g., the brain, thus requiring only a fraction of the systemic dose (see, e. g. , Goodson, in Medical Applications of Controlled Release, supra, vol. 2, pp. 115-138 (1984)). Other controlled-release systems discussed in the review by Langer (Science 249: 1527-1533 (1990)) can be used.

The term carrier refers to a diluent, adjuvant or excipient, with which a conjugate of the invention is administered. Such pharmaceutical carriers can be liquids, such as water and oils, including those of petroleum, animal, vegetable or synthetic origin, such as peanut oil, soybean oil, mineral oil, sesame oil and the like. The carriers can be saline, gum acacia, gelatin, starch paste, talc, keratin, colloidal silica, urea, and the like. In addition, auxiliary, stabilizing, thickening, lubricating and coloring agents can be used. In one embodiment, when administered to an animal, the Compounds of the Invention or compositions and pharmaceutically acceptable carriers are sterile. Water is a preferred carrier when the Compounds of the Invention are administered intravenously. Saline solutions and aqueous dextrose and glycerol solutions can also be employed as liquid carriers, particularly for injectable solutions. Suitable pharmaceutical carriers also include excipients such as starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like. The present compositions, if desired, can also contain minor amounts of wetting or emulsifying agents, or pH buffering agents.

The present compositions can take the form of solutions, suspensions, emulsion, tablets, pills, pellets, capsules, capsules containing liquids, powders, sustained-release formulations, suppositories, emulsions, aerosols, sprays, suspensions, or any other form suitable for use.

A general reference can be made to "Remington's Pharmaceutical Sciences" by E. W. Martin.

The following examples further illustrate the present invention.

### Example 1

### Preparation of cryptophycin-55-cilycinate ADC upon partial reduction of the antibody inter-chain disulfide bonds with TCEP (tris(2-carboxyethyl)phosphine) method:

From a freshly prepared PBS-EDTA-buffer pH 7.2 a TCEP-PBS-EDTA-solution [3 mM TCEP (Sigma-Aldrich) in 100 uL of PBS-EDTA-solution] and a mAb-PBS-EDTA-solution (9 mg/mL Trastuzumab in 0.8 mL PBS-EDTA-solution; 62 uM Trastuzumab) were prepared. The TCEP-PBS-EDTA-solution was added to the mAb-PBS-EDTA-solution and the resulting solution was incubated at 25°C for 60 min. Final concentrations of the reagents were as follows: 8 mg/mL reduced Trastuzumab (55 uM), 290 uM TCEP.

Then desalting on a HiTrap Desalting Sephadex G25-5 mL (GE Healthcare/Amersham) column pre-equilibrated in PBS-EDTA-buffer pH7.2 was performed using an Äkta instrument, and fractions of 0.25 mL volume were collected in PBS-EDTA-buffer. All fractions were tested for their protein-content (2 uL of each fraction was added to 200 µL Bradford-solution). The protein-containing fractions were combined and the antibody concentration was re-determined by Bradford assay resulting 3.3 mg/mL (22.8 uM) with a recovery of 5 mg of total antibody (70% recovery). The concentration of free -SH was determined using Elmann's reagent DTNB (5,5'-dithiobis-(2-nitrobenzoic acid), Sigma-Aldrich/Fluka) and a standard curve with L-Cys (40-50-100-200-400-800 uM). Total -SH concentration was 68.24 uM with an average of three free -SH per antibody.

Partial reduction of inter-chain disulfide bonds was repeated as described above and a solution of Maleimide-PEG-Val-Cit-Pro-Gly-Cryptophycin-55-glycinate (1.5 mM in DMSO; 100 uL) was directly added drop-wise to the sample (900 uL) under continuous agitation for 2h at 4°C, reaching a final concentration of 150 uM in 10% DMSO, such to obtain a 1:1 molar ratio relative to the free -SH groups and a 3:1 molar ratio relative to the antibody (antibody final concentration was 50 uM, equivalent to 150 uM of total free SH). In parallel a S-S reduced antibody control sample has been left unconjugated as control. At the end of the incubation time, each sample was immediately loaded onto a HiTrap Sephadex G25 column and desalting performed as above. Bradford and Elmann assays were performed on pooled fractions as described above and the results are summarized below.

| **Samples** | **FXN pools** | **mg/ml IgG** | **µM IgG** | **Total mg IgG** | **Yield** | **µM free SH** | **SH/IgG** |
|---|---|---|---|---|---|---|---|
| ADC | 2-7: 1.25 ml | 3 | 20.9 | 3.75 | 52% | 8.6 | 0.5 |
| C | 7-13: 875 µL | 1.65 | 11.4 | 1.44 | 40% | 30.5 | 2.7 |

Under the selected immunoconjugation reaction conditions the average number of SH/lgG dropped from 2.7 to 0.5, thus suggesting that binding to the drug linker occurred with a drug-antibody ratio (DAR) of about 2.

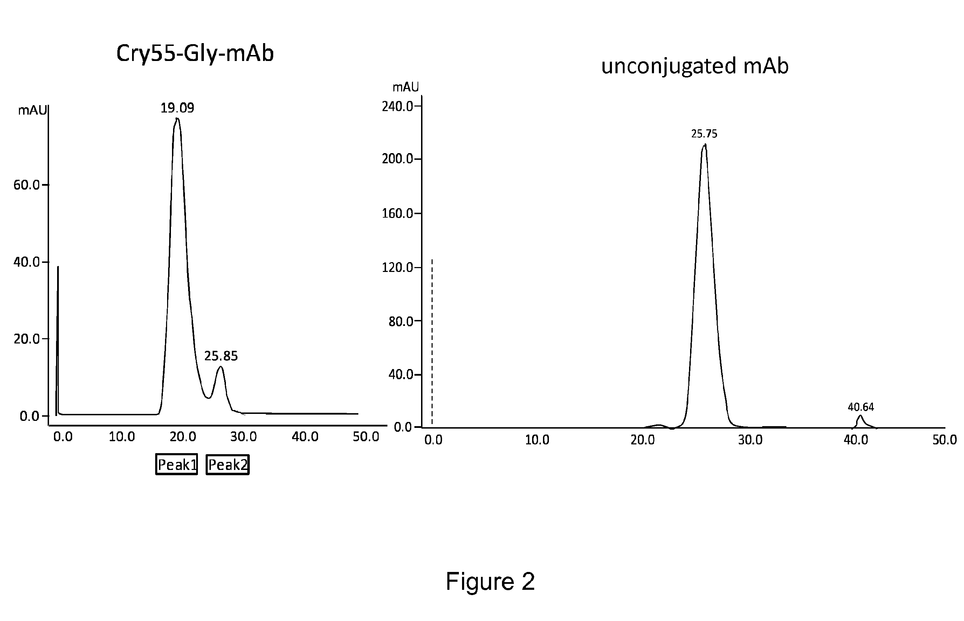

Then preparative gel exclusion chromatography was performed using an Äkta instrument. 500 uL of the cryptophycin-55-glycinate-conjugated and unconjugated mAb samples were loaded onto a Superdex 200 10/300 GL column (GE Healthcare/Amersham) using PBS-EDTA pH7.2 as the chromatographic buffer and applying a flow-rate of 0.5 ml/min. 250 uL-fractions were collected. Purification of the drug linker-conjugated antibody held to two protein peaks, peak 2 corresponding to the monomeric form and peak 1 corresponding to an oligomeric form of the cryptophycin-55-glycinate-conjugated antibody, as assessed by comparison with suitable protein MW standards (Figure 2, left panel). On the contrary, the unconjugated mAb sample resulted in a single peak, corresponding to the monomeric form of the antibody ("mAb control" (Figure 2, right panel). Peak fractions were pooled and protein concentration determined by Bradford assay. Results are summarized below.

| **Samples** | **FXN pools** | **mg/ml IgG** | **µM IgG** | **Total mg IgG** | **Yield** |
|---|---|---|---|---|---|
| Peak 1 | 32-44: 3.25 ml | 0.14 | 0.96 | 0.46 | 53% |
| Peak 2 | 47-55: 2.25 ml | 0.02 | 0.14 | 0.05 | 5.8% |
| Reduced mAb | 46-56: 2.75 ml | 0.34 | 2 | 0.93 | 63% |

### Example 2

### Preparation of the cryptophycin-55-glycinate ADC upon partial reduction of the antibody inter-chain disulfide bonds with 2-MEA (2-Mercaptoethylamine·HCl) method:

From a freshly prepared PBS-EDTA-buffer (137 mM NaCl; 2.7 mM KCl; 10 mM Na2HPO4; 2 mM KH2PO4; 10 mM EDTA; pH = 7.2) a 2-MEA-PBS-EDTA-solution (6 mg 2-MEA in 100 uL PBS-EDTA-solution) and a mAb-PBS-EDTA-solution (10 mg Trastuzumab in 1 mL PBS-EDTA-solution; 68.7 uM Trastuzumab) were prepared. The 2-MEA-PBS-EDTA-solution (300 uL) was added to mAb-PBS-EDTA-solution (1 mL) and the resulting solution was incubated at 37°C for 90 min. Final concentrations of the reagents were as follows: 7.7 mg/mL reduced Trastuzumab (53 uM), 13.8 mg 2-MEA (122 mM).

Then desalting on a HiTrap Desalting Sephadex G25 5 cm column (PBS-EDTA-buffer) was performed and fractions of 0.5 mL volume were collected. All fractions were visually tested for their protein-content (2 µL of each fraction was added to 200 µL Bradford-solution). The protein-containing fractions were combined and a solution of Maleimide-PEG-Val-Cit-Pro-Gly-Cryptophycin-55-glycinate (1.82 mM in DMSO; 150 uL) was added. Incubation at 6°C for 2 h was followed by concentration of the solution to a volume of less than 1.5 mL. Purification on Äkta Purifier (Superdex 200 highload 16/60 column; PBS-EDTA-buffer) lead to two fractions which were concentrated to mAb-Cry-55-linker-Peak1 (1.54 µg/µL) and mAb-Cry-55-linker-Peak2 (0.32 µg/µL). Peak 1 and 2 corresponded to the two antibody aggregation states described above.

### Example 3

### Cell viability assays

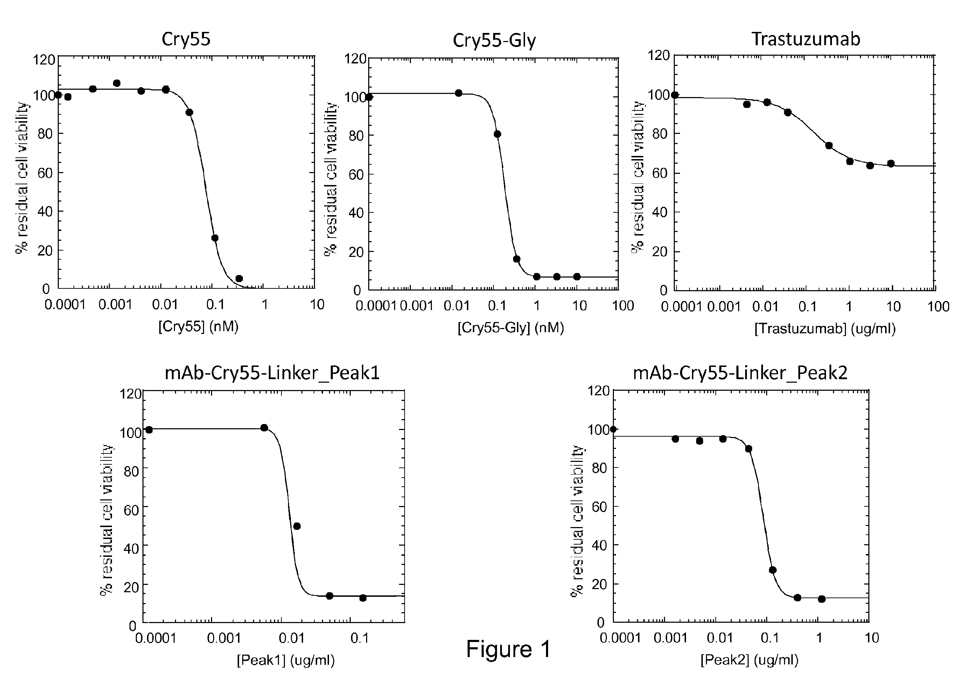

Tumor cell lines (breast carcinoma cell lines SKBR-3 and MCF7 and colon carcinoma cell line HCT116) were obtained from American Type Culture Collection and were grown according to standard protocols. SKBR-3 cells are known to express high Her2 levels and therefore are trastuzumab-sensitive, while MCF7 and HCT116 cells show low Her2 expression and therefore are trastuzumab-resistant. The effects of the cryptophycin-55-glycinate ADC conjugate on tumor cell viability were assessed using the CellTiter-Glo® Luminescent Cell Viability Assay (Promega), according to the manufacturer protocol. To be able to detect a difference between the unconjugated trastuzumab and the trastuzumab ADC, we had previously identify experimental conditions (concentration ranges, incubation time) under which trastuzumab only affected SKBR-3 cell viability by about 30%, while free cryptophycin 55 was equally effective on all tumor cell lines. Cells were plated in black-walled 96-well plates (5000 cells per well for SKBR-3 and MCF7; 2000 cells per well for HCT116) and allowed to adhere overnight at 37°C in a humidified atmosphere of 5% CO2. Medium was then removed and replaced by fresh culture medium containing increasing concentrations of trastuzumab ADC, unconjugated trastuzumab, or free cryptophycin analogs (CRY-55, CRY55-Gly), and the cells were incubated for 96h at 37°C in 5% CO2. Five to eight triplicate datapoints were recorded for each experimental condition. At the end of the incubation luminescence was measured using a Perkin Elmer Wallac 1450 MicroBeta TriLux detector. Compound cytotoxicity was evaluated in comparison to cells treated with PBS (antibodies) or 0.1 % DMSO (compounds). CC₅₀ values were calculated by fitting viability data with a four-parameter logistic equation using a Kaleidagraph software. Residual cell viability plots obtained upon treatment of SK-BR3 cells with the different compounds are shown in Figure 1. The novel cryptophycin-55-glycinate ADC (Figure 1, lower panels) shows a much higher cytotoxic effect than naked trastuzumab (Figure 1, upper-right panel) on this Her2-positive breast carcinoma cell line, which is comparable with that shown by cryptophicin-55 and cryptophycin-55-glycinate (Figure 1, upper-left and -medium panels, respectively). Different from cryptophycin-55 and cryptophycin-55-glycinate, the ADC cytotoxic effect is highly selective for the HER2-positive SK-BR3 cells compared to the Her2-low MCF7 and HCT116 cell lines (Table I).

**Table 1: CC₅₀ values**

| | **Cell line** | | |
|---|---|---|---|
| | ***SK-BR3*** | ***MCF7*** | ***HCT116*** |
| **Compound** | CC₅₀ | CC₅₀ | CC₅₀ |
| Cry-₅₅ | 0.076 nM | 0.18 nM | 0.1 nM |
| Cry-55-Linker | 2.6 nM | 6.1 nM | 4.4 nM |
| Cry-55-Gly | 0.09 nM | 0.25 nM | 0.19 nM |
| Trastuzumab | 0.15 µg/mL | inactive | inactive |
| Trastuzumab reduced | 0.08 µg/mL | inactive | inactive |
| Cry-55-Gly-mAb_Peak1 | 0.01 µg/mL | inactive | inactive |
| Cry-55-Gly-mAb_Peak2 | 0.05 µg/mL | inactive | inactive |

### Example 4

### Preparation of cryptophycin-52

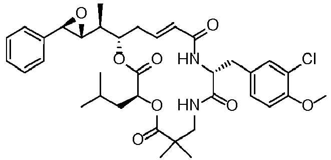

M = 669.20 g·mol⁻¹ C₃₆H₄₅ClN₂O₈

Cryptophycin-52 was prepared according to a route published by Weiss C, et al (2012) Beilstein Journal of Organic Chemistry 8:2060-6 and Weiss C, et al (2013) Natural Product Reports 30:924-40.

### Preparation of Cryptophycin-55

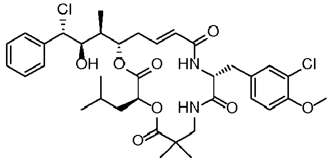

M = 705.67 g·mol ⁻¹ C₃₆H₄₆Cl₂N₂O₈

Cryptophycin-52 (40 mg; 60 µmol; 1 eq) was dried in high vacuum and subsequently dissolved in abs. DCM (1 mL). The solution was cooled to -50°C and HCl (4 M in dioxane; 74.7 µL; 0.30 mmol; 5 eq) was added. After stirring for 2 h the solution was allowed to warm to room temperature (rt) and the volatiles were removed under reduced pressure. Column chromatography (SiO₂; PE/EtOAc 1:3) yielded Cryptophycin-55 (43 mg; 60 µmol; quant.) as colorless solid.
**R_{f}** (PE/EtOAc 1:3) = 0.27;
**¹H-NMR** (600 MHz, Chloroform-d, TMS) δ [ppm] = 7.41 - 7.34 (m, 5H, uA-C*^{ar}*H); 7.21 (m, 1 H, uC-NH); 7.22 (d, 1 H, *J* = 2.2 Hz, uB-C^{2'}H); 7.08 (dd, 1 H, *J* = 8.3/2.1 Hz, uB-C^{6'}H); 6.86 (d, 1 H, *J* = 8.4 Hz, uB-C^{5'} H); 6.78 (ddd, 1H, *J* = 15.1/10.6/4.4 Hz, uA-C*^{β}*H); 5.78 (dd, 1H, *J* = 15.1/1.8 Hz, uA-C*^{α}*H); 5.52 (d, 1H, *J* = 7.9 Hz, uB-NH); 5.16 (ddd, 1 H, *J* = 10.6/8.2/2.0 Hz, uA-C*^{δ}*H); 4.93 (dd, 1 H, *J =* 10.2/3.5 Hz, uD-C*^{α}*H); 4.75 (ddd, 1 H, *J* = 7.9/7.7/5.2 Hz, uB-C*^{α}*H); 4.65 (d, 1 H, *J* = 9.6 Hz, uA-C*^{η}*H); 4.01 (dd, 1 H, *J* = 9.7/1.9 Hz, uA-C*^{ξ}*H); 3.88 (s, 3H, uB-OCH₃); 3.38 (dd, 1H, *J* = 13.4/8.2 Hz, uC-CH^{A}H^{B}NH); 3.18 (dd, 1H, *J* = 13.5/3.8 Hz, uC-CH^{A}H^{B}NH); 3.14 (dd, 1H, *J* = 14.5/5.2 Hz, uB-C*^{β}*H^{A}H^{B}); 3.06 (dd, 1 H, *J* = 14.4/7.6 Hz, uB-C*^{β}*H^{A}H^{B}); 2.70 (m, 1H, uA-C*^{γ}*H^{A}H^{B}); 2.49 (m, 1H, uA-C*^{ε}*H); 2.38 (ddd, 1H, *J* = 14.5/10.9/10.9 Hz, uA-C*^{γ}*H^{A}H^{B}); 1.78 (ddd, 1H, *J* = 14.0/4.5/4.0 Hz, uD-C*^{β}*H^{A}H^{B}); 1.72 (m, 1H, uD-C*^{γ}*H); 1.43 (ddd, 1H, *J* = 14.0/8.7/3.5 Hz, uD-C*^{β}*H^{A}H^{B});1.23 (s, 3H, uC-C(CH₃)₂); 1.17 (s, 3H, uC-C(CH₃)₂); 1.04 (d, 3H, *J* = 6.9 Hz, uA-C*^{ε}*HCH₃); 0.93 (d, 3H, *J* = 1.4 Hz, uD-C*^{δ}*H₃); 0.92 (d, 3H, *J =* 1.2 Hz, uD-C*^{δ}*H₃).

### Preparation of Cryaptophycin-55-glycinate trifluoroacetate

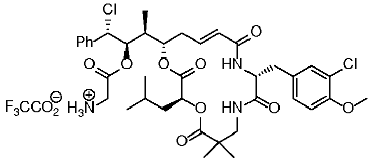

M = 875.28 g·mol⁻¹ C₄₀H₅₀Cl₃F₃N₃O₁₁

Preparation of Cryptophycin-55-glycinate trifluoroacetate was performed according to Liang J, et al (2005) Investigational New Drugs 213-224 whereas the synthesis was done without purification after the first reaction step. Additionally, the product was not purified with column chromatography but RP-HPLC to yield the Cryptophycin-55-glycinate trifluoroacetate salt.

**¹H-NMR** (500 MHz, Chloroform-d, TMS) δ [ppm] = 7.37 - 7.32 (m, 3H, uA-C*^{ar}*H); 7.29 (dd, 2H, *J* = 7.3/2.3 Hz, uA-C*^{ar}*H); 7.22 (m, 1 H, uC-NH); 7.20 (d, 1 H, *J* = 2.2 Hz, uB-C^{2'}H); 7.06 (dd, 1 H, *J =* 8.4/2.2 Hz, uB-C^{6'}H); 6.84 (d, 1 H, *J* = 8.5 Hz, uB-C^{5'}H); 6.60 (bs, 1 H, uB-NH); 6.53 (ddd, 1 H, *J* = 15.2/10.9/4.2 Hz, uA-C*^{β}*H); 5.74 (dd, 1H, *J* = 15.3/1.8 Hz, uA-C*^{α}*H); 5.45 (dm, 1H, *J* = 10.1 Hz, uA-C*^{ξ}*H); 4.94 (dd, 1H, *J =* 10.4/2.9 Hz, uD-C*^{α}*H); 4.85 - 4.79 (m, 2H, uA-C*^{δ}*H/uA-C*^{η}*H); 4.54 (ddd, 1H, *J =* 8.1/8.1/5.3 Hz, uB-C*^{α}*H); 3.87 (s, 3H, uB-OCH₃); 3.63 (d, 1 H, *J* = 16.7 Hz, Gly-CH^{A}H^{B}); 3.30 (m, 1 H, uC-C*^{β}*H^{A}); 3.20 (m, 1 H, uC-C*^{β}*H^{B} 3.16 - 3.06 (m, 2H, Gly-CH^{A}H^{B}/uB-C*^{β}*H^{A}) ; 2.91 (dd, 1H, *J* = 14.5/8.6 Hz, uB-C*^{β}*H^{B}); 2.64 (m, 1 H, uA-C*^{ε}*H); 2.53 (m, 1H, uA-C*^{γ}*H^{A}); 2.22 (m, 1H, uA-C*^{γ}*H^{B}); 1.93 (m, 1H, uD-C*^{β}*H^{A}); 1.70 (m, 1H, uD-C*^{γ}*H); 1.65 (m, 1H, uD-C*^{β}*H^{B}); 1.18 (s, 3H, uC-CH₃); 1.12 (s, 3H, uC-CH₃); 1.00 (d, 3H, *J* = 7.2 Hz, uA-C^{ε}CH₃); 0.98 (d, 3H, J = 6.7 Hz, CH₃); 0.93 (d, 3H, *J* = 6.5 Hz, CH₃).

### Preparation of self-immolative linker

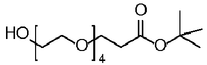

M = 322.39 g·mol⁻¹ C₁₅H₃₀O₇

*tert-*Butyl-15-hydroxy-4,7,10,13-tetraoxapentadecanoate was synthesized according to a procedure published in Seitz O, et al (1997) Journal of Organic Chemistry 62:813-826.

To a solution of tetraethyleneglycol (40.61 mL; 45.64 g; 235 mmol) in abs. THF (125 mL) a piece of sodium (1/4 cm) was added. After the sodium had reacted completely, *tert*-butylacrylate (11.98 mL; 10.57 g; 82.5 mmol) was added dropwise over 20 min and the resulting solution was stirred at rt overnight. The pH was adjusted to 7-8 with NaOH solution (1N) and the solvents were removed in vacuum. The residue was dissolved in sat. NaCl solution (75 mL) and extracted with EtOAc (3x100 mL). The combined organic layers were dried over MgSO₄ and after evaporation of the solvent *tert*-butyl-15-hydroxy-4,7,10,13-tetraoxapentadecanoate (21.87 g; 67.8 mmol; 82% based on *tert*-butylacrylate) was obtained as colorless oil.
**¹H-NMR** (500 MHz, Chloroform-d, TMS) δ [ppm] = 3.77 - 3.57 (m, 18H; OCH₂); 3.01 (bs, 1H, OH); 2.51 (t, 2H, *J* = 6.6Hz, CH₂COO*^{t}*Bu); 1.45 (s, 9H, C(CH₃)₃);
**¹³C{¹H}-NMR** (126 MHz, Chloroform-d, TMS) δ [ppm] = 171.1 (COO); 80.7 (C(CH₃)₃); 72.6/70.8/70.7/70.6/70.5/67.0 (OCH₂); 61.9 (HOCH₂); 36.4 (CH₂CO); 28.2 (C(CH₃)₃). 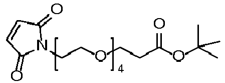 M = 401.45 g·mol⁻¹ C₁₉H₃₁NO₈

*tert*-Butyl-15-maleimido-4,7,10,13-tetraoxapentadecanoate was synthesized according to Warnecke A, (2002) Dissertation. Ph₃P (1.09 g; 4.14 mmol; 1 eq) was dissolved in abs. THF (25 mL) and the resulting solution was cooled to -70°C. DIAD (0.8 mL; 4.14 mmol; 1 eq) was added over 1 min and the yellow solution was stirred for 5 min at -70°C. Then *tert*-butyl-15-hydroxy-4,7,10,13-tetraoxapentadecanoate (2.0 g; 6.20 mmol; 1.5 eq) was added and the solution was stirred for additional 5 min prior to the addition of maleimide (401 mg; 4.14 mmol; 1 eq). After additional stirring for 5 min at -70°C the cooling bath was removed and the mixture was allowed to warm up to rt. Stirring was continued for 18 h at rt, then the solvent was removed in vacuo and the oily residue was purified by column chromatography (SiO₂; PE/EtOAc 1:3). *tert*-Butyl-15-maleimido-4,7,10,13-tetraoxapentadecanoate (0.61 g; 1.51 mmol; 36%) was obtained as colorless oil. **R_{f}** (PE/EtOAc 1:3) = 0.27;
**R_{f}** (PE/EtOAc 1:1) = 0.19;
**¹H-NMR** (500 MHz, Chloroform-d, TMS) δ [ppm] = 6.70 (s, 2H, CH=CH); 3.71 (m, 4H, CH₂); 3.66 - 3.53 (m, 14H, CH₂); 2.49 (t, 2H, *J* = 6.6 Hz, CH₂COO*^{t}*Bu); 1.44 (s, 9H, C(CH₃)₃);
**¹³C{¹H}-NMR** (126 MHz, Chloroform-d, TMS) δ [ppm] = 171.0 (CH₂COO*^{t}*Bu); 170.8 (COCH=CHCO); 134.3 (CH=CH); 80.6 (C(CH₃)₃); 70.8/70.73/70.70/70.65/70.5/70.2/68.0/67.1 (OCH₂); 37.3 (NCH₂); 36.4 (CH₂COO*^{t}*Bu); 28.3 (C(CH₃)₃). 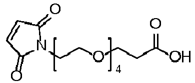 M = 345.35 g·mol⁻¹ C₁₅H₂₃NO₈

15-Maleimido-4,7,10,13-tetraoxapentadecanoic acid was prepared according to Warnecke A, (2002) Dissertation. *tert*-Butyl-15-maleimido-4,7,10,13-tetraoxapentadecanoate (1.74 g; 4.33 mmol) was dissolved in abs. DCM (8 mL) and TFA (8 mL) was added. The solution was stirred at rt for 1 h. Next the volatile compounds were removed in vacuo and the residue was again dissolved in DCM (20 mL). Amberlyst A-21 (6 g) was added to the solution and everything was stirred 1 h at rt. Next the solid was filtered off and the filtrate was concentrated to dryness. 15-Maleimido-4,7,10,13-tetraoxapentadecanoic acid (1.46 g; 4.23 mmol; 98%) was obtained as dark yellow oil.
**¹H-NMR** (500 MHz, Chloroform-d, TMS) δ [ppm] = 9.38 (bs, 1H, COOH); 6.71 (s, 2H, CH=CH); 3.75 (dt, 4H, *J =* 21.6/5.8 Hz, NCH₂/CH₂); 3.68 - 3.58 (m, 14H, CH₂); 2.63 (t, 2H, *J =* 6.1 Hz, CH₂COOH). 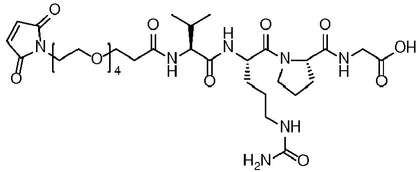 M = 755.81 g·mol⁻¹ C₃₃H₅₃N₇O₁₃

Maleimide-PEG-Val-Cit-Pro-Gly was synthesized according to standard Fmoc SPPS [Fields G and Noble R L, (2009) International Journal of Peptide and Protein Research 33,3:161-214].

See Table for details.

| Step | Substance | Volume | Mass | Amount of substance | Equivalents | Reaction time |
|---|---|---|---|---|---|---|
| 1 | Barlos resin | | 1.0 g | | | |
| | Fmoc-Gly | | 1.43 g | 4.8 mmol | 3 eq | |
| | DIPEA | 1.22 mL | 0.93 g | 7.2 mmol | 4.5 eq | |
| | abs. DCM | 5 mL | | | | 3h |
| 2 | Fmoc-L-Pro | | 1.18 g | 3.48 mmol | 3 eq | |
| | DIPEA | 0.79 mL | 0.60 g | 4.64 mmol | 4 eq | |
| | TBTU | | 1.12 g | 3.48 mmol | 3 eq | |
| | HOBt·H₂O | | 0.47 g | 3.48 mmol | 3 eq | |
| | DMF | 7 mL | | | | 3h |
| 3 | Fmoc-L-Cit | | 1.38 g | 3.48 mmol | 3 eq | |
| | DIPEA | 0.79 mL | 0.60 g | 4.64 mmol | 4 eq | |
| | TBTU | | 1.12 g | 3.48 mmol | 3 eq | |
| | HOBt·H₂O | | 0.47 g | 3.48 mmol | 3 eq | |
| | DMF | 10 mL | | | | 3h |
| 4 | Fmoc-L-Cit | | 0.92 g | 2.32 mmol | 2 eq | |
| | DIPEA | 0.53 mL | 0.41 g | 3.13 mmol | 2.7 eq | |
| | TBTU | | 0.75 g | 2.32 mmol | 2 eq | |
| | HOBt·H₂O | | 0.31 g | 2.32 mmol | 2 eq | |
| | DMF | 7 mL | | | | overnight |
| 5 | Fmoc-L-Val | | 1.18 g | 3.48 mmol | 3 eq | |
| | DIPEA | 0.79 mL | 0.60 g | 4.64 mmol | 4 eq | |
| | TBTU | | 1.12 g | 3.48 mmol | 3 eq | |
| | HOBt·H₂O DMF | 7 mL | 0.47 g | 3.48 mmol | 3 eq | 2h |
| 6 | Maleimide functionalized | | 0.80 g | 2.32 mmol | 2 eq | |
| | PEG-linker | | | | | |
| | DIPEA | 0.79 mL | 0.60 g | 4.64 mmol | 4 eq | |
| | TBTU | | 0.75 g | 2.32 mmol | 2 eq | |
| | HOBt·H₂O | | 0.31 g | 2.32 mmol | 2 eq | |
| | DMF | 7 mL | | | | overnight |

After cleavage from the resin the residue was purified by RP-HPLC and Maleimide-PEG-Val-Cit-Pro-Gly (0.61 g; 0.81 mmol; 70%) was received as colorless lyophilisate.

**¹H-NMR** (500 MHz, DMSO-*d*₆) δ [ppm] = 12.24 (bs, 1H, COOH); 8.10 (dd, 1H, *J =* 6.1/5.8 Hz, Gly-NH); 8.06 (d, 1 H, *J* = 7.3 Hz, Cit-NHCO); 7.81 (d, 1 H, *J =* 8.9 Hz, Val-NHCO); 7.02 (s, 2H, CH Maleimide); 6.02 (bm, 1 H, Cit-NH); 4.43 (dd, 1 H, *J* = 13.4/7.5 Hz, Cit-C*^{α}*H); 4.31 (dd, 1 H, *J =* 8.3/3.9 Hz, Pro-C*^{α}*H); 4.20 (dd, 1H, *J* = 8.8/6.7 Hz, Val-C*^{α}*H); 3.78 (dd, 1H, *J* = 17.5/6.1 Hz, Gly-CH^{A}); 3.69 (m, 1H, Pro-C*^{γ}*H^{A}); 3.68 (dd, 1 H, *J* = 17.5/5.8 Hz, Gly-CH^{B}); 3.62 - 3.53 (m, 5H, PEG-CH₂ /Pro-C*^{γ}*H⁶); 3.53 - 3.41 (m, 16H, PEG-CH₂); 3.00 - 2.90 (m, 2H, Cit-CH₂NH); 2.45 (m, 1H, PEG-CH^{A}H^{B}CONH); 2.34 (m, 1H, PEG-CH^{A}H^{B}CONH); 2.01 (m, 1H, Pro-C*^{β}*H^{A}); 1.97 - 1.87 (m, 2H, Val-C*^{β}*H/Pro-C*^{δ}*H^{A}); 1.87 - 1.79 (m, 2H, Pro-C*^{β}*H^{B}/Pro-C*^{γ}*H^{B}); 1.67 (m, 1H, Cit-C*^{β}*H^{A}); 1.49 (m, 1H, Cit-C*^{β}*H^{B}); 1.44 - 1.36 (m, 2H, Cit-C*^{γ}*H₂); 0.81 (d, 3H, *J* = 6.8 Hz, Val-CH₃); 0.79 (d, 3H, *J* = 6.8 Hz, Val-CH₃);
**¹³C{¹H}-NMR** (126 MHz, DMSO-*d*₆) δ [ppm] = 172.0 (Pro-CO); 171.3 (COOH); 171.0 (Val-CO); 170.9 (Maleimide-CO); 170.1 (Cit-CONH); 170.0 (PEG-CONH); 158.9 (Cit-CONH₂); 134.6 (Maleimide-CH); 69.8/69.77/69.72/69.66/69.5/69.4/67.0 (PEG-CH₂); 59.3 (Pro-C*^{α}*); 57.1 (Val-C*^{α}*); 50.2 (Cit-C*^{α}*); 46.8 (Pro-C*^{γ}*); 40.6 (Gly-CH₂); 30.7 (Val-C*^{β}*) 29.2 (Pro-C*^{β}*); 28.3 (Cit-C*^{β}*); 26.1 (Cit-C*^{γ}*; 24.4 (Pro-C*^{γ}*; 19.2 (Val-C*^{γ}*); 18.1 (Val-C*^{γ}*);
**HRMS** (ESI-FT-ICR):

| | | |
|---|---|---|
| Measured [m·z⁻¹] = | 778.35962 | [C₃₃H₅₃N₇O₁₃+Na]⁺ |
| Calculated [m·z⁻¹] = | 778.35936 | [C₃₃H₅₃N₇O₁₃+Na]⁺. |

### Cryptophycin-55-glycinate Peptide for Maleimide Conjugation

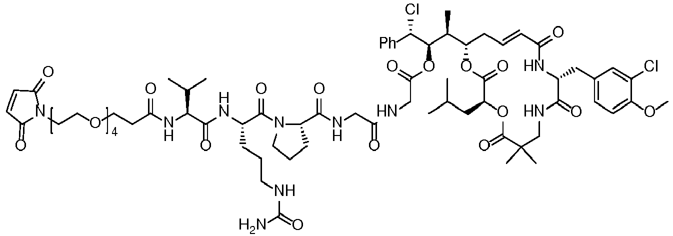

M = 1500.51 g·mol⁻¹ C₇₁H₁₀₀Cl₂N₁₀O₂₁

Cryptophycin-55-glycinate trifluoroacetate (10 mg; 13.1 µmol; 1 eq) and Maleimide-PEG-Val-Cit-Pro-Gly (29.7 mg; 39.3 µmol; 3 eq) were dissolved in abs. DMF (0.5 mL). A solution of DIPEA (10 µL; 52.4 µmol; 4 eq), TBTU (12.6 mg; 39.3 µmol; 3 eq) and HOBt·H₂O (5.3 mg; 39.3 µmol; 3 eq) in abs. DMF (0.5 mL) was slowly added. After stirring over night at rt again DIPEA, TBTU and HOBt·H₂O (same amounts as mentioned above) were added. It was stirred again for further 2 h, then the reaction solution directly purified by RP-HPLC. Maleimide-PEG-Val-Cit-Pro-Gly-Cryptophycin-55-glycinate (11.8 mg; 8 µmol; 60%) was achieved as colorless lyophilisate.
**HRMS** (ESI-FT-ICR):

| | | |
|---|---|---|
| Measured [m·z⁻¹] = | 1521.63460 | [C₇₁H₁₀₀Cl₂N₁₀O₂₁+Na]⁺ |
| Calculated [m·z⁻¹] = | 1521.63338 | [C₇₁H₁₀₀Cl₂N₁₀O₂₁+Na]⁺. |

## Claims

1. A conjugate of formula (I) 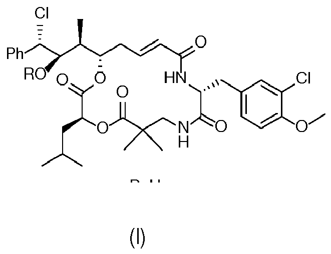 wherein R is -CO-CH₂-X-(A)ₙ-B
wherein X is selected from the group consisting of NR^{a}, wherein R^{a} is selected from a group consisting of H, C₁-C₁₀ alkyl or
X is -O;
A is a self-immolative linker,
n is 0 or 1;
B is selected from the group consisting of a peptide, a polypeptide/protein, and an antibody and the pharmaceutically acceptable salts thereof.

2. The conjugate according to claim 1, wherein the antibody is a monoclonal antibody, or a nanobody.

3. The conjugate of claim 2, wherein the nanobody is selected from the group consisting of single-domain antibody and camelid antibody.

4. The antibody drug conjugate (ADC) according to claim 2, wherein the monoclonal antibody is selected from the group consisting of trastuzumab, gemtuzumab, brentuximab, rituximab, cetuximab, panitumumab, ofatumumab, obinutuzumab, pertuzumab.

5. The conjugate according to claim 1, where the peptide B binds to somatostatin receptors

6. The conjugate (PDC) according to claim 5 where the peptide is selected from the group consisting of octreotide, pasireotide or lanreotide.

7. The conjugate according to any one of claims claim 1-6, wherein the self-immolative linker A is a moiety of formula (II) 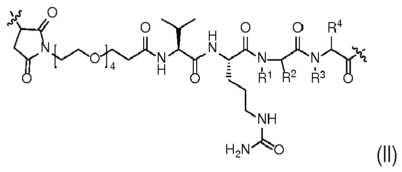 wherein R¹ is selected from the group consisting of H, (C₁-C₁₀) alkyl R², optionally together with R¹, is the residue of an amino acid side chain, R³ is selected from the group consisting of H, (C₁-C₁₀) alkyl R⁴, optionally together with R³ is the residue of an amino acid side chain.

8. The conjugate according to claim 7, which has formula (III) 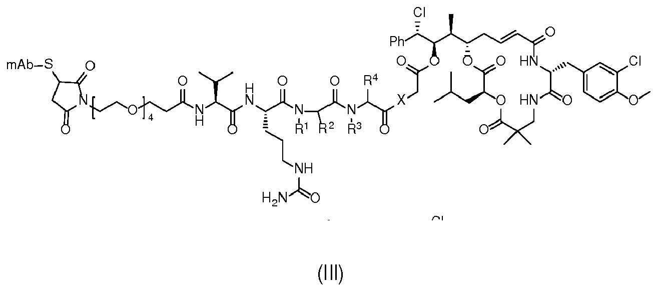 wherein R¹, R², R³ and R⁴ are as defined above, X is selected from the group consisting of NH, N- (C₁-C₁₀)- Alkyl, O; mAb represents a monoclonal antibody or a nanobody, or a peptide or a polypeptide.

9. The conjugate according to claim 7 of formula (IV),

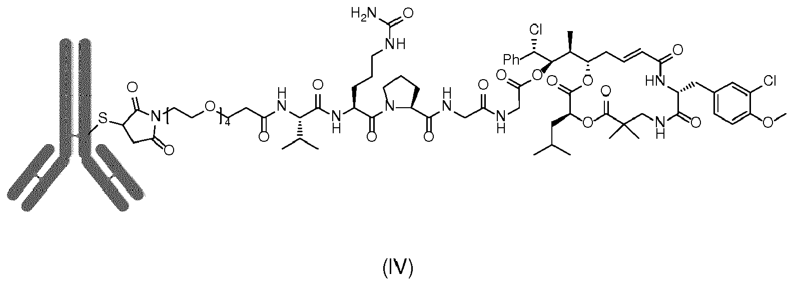

10. The conjugate according to claim 7 of formula (V) 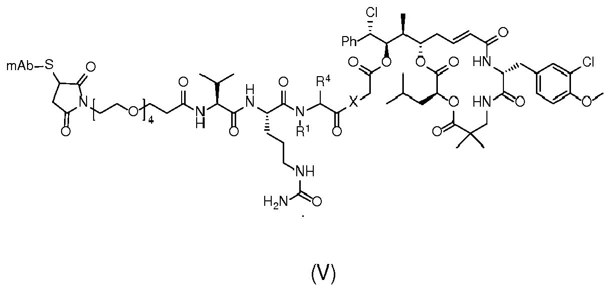 wherein R¹ is selected from the group consisting of H, (C₁-C₁₀) alkyl R⁴, optionally together with R¹, is the residue of an amino acid side chain; X is selected from the group consisting of NH, N- (C₁-C₁₀), Alkyl O; mAb represents a monoclonal antibody, a peptide or a polypeptide.

11. A pharmaceutical composition comprising a conjugate of any one of claims 1-10 as active ingredient in admixture with at least one pharmaceutically acceptable vehicle and/or excipient.

12. The conjugate of any one of claims 1-10 for use as a medicament.

13. The conjugate for use according to claim 12 for the therapeutic treatment of a disease selected from the group consisting of cancer or an autoimmune disease or an infective disease.

14. The conjugate of any one of claims 1-10 for use as a diagnostic.

15. The conjugate for use according to claim 14, for the diagnosis of a disease selected from the group consisting of cancer or an autoimmune disease or an infective disease.
